# EUROPÄISCHE PATENTSCHRIFT

(11) **EP 2 255 181 B1**
(45) Veröffentlichungstag und Bekanntmachung des Hinweises auf die Patenterteilung: **23.11.2016**
(21) Anmeldenummer: 09722158.4
(22) Anmeldetag: 13.03.2009
(51) Int. Cl.: G01N 33/00, G01W 1/02, G01K 3/06, G01K 13/02, G01N 27/04

(54) **MESSVORRICHTUNG FÜR FEUCHTE UND TEMPERATUR EINES STRÖMENDEN MEDIUMS**
DEVICE FOR MEASURING HUMIDITY AND TEMPERATURE OF A FLOWING MEDIUM
DISPOSITIF DE MESURE DE L HUMIDITÉ ET DE LA TEMPÉRATURE D UN FLUIDE EN ÉCOULEMENT

(30) Priorität: 19.03.2008 DE 102008014926; 24.06.2008 DE 102008029793
(43) Veröffentlichungstag der Anmeldung: 01.12.2010
(73) Patentinhaber: Epcos AG, 81669 München (DE)
(72) Erfinder: BOHL, Benjamin, 10407 Berlin (DE); BALZER, Peter, 10179 Berlin (DE)
(74) Vertreter: Epping - Hermann - Fischer
(86) Internationale Anmeldenummer: PCT/EP2009/053026
(87) Internationale Veröffentlichungsnummer: WO 2009/115471

(56) Entgegenhaltungen:
- EP-A- 1 696 184
- WO-A-02/16908
- WO-A-2007/079516
- DE-A1- 10 024 490
- DE-A1- 10 152 777
- DE-A1- 10 336 690
- DE-B3-102006 021 528
- FR-A- 2 750 771

## Beschreibung

Es wird eine Messvorrichtung zur Messung des Feuchtigkeitsgehalts eines strömenden Mediums angegeben.

In der Druckschrift DE 102006021528 B3 ist ein Fühler zur Messung der Temperatur eines Luftstroms beschrieben. Ferner offenbart die Druckschrift US 4 480 334 eine Messvorrichtung mit einem Fenchtigkeitssensor im verengten Bereich eines Kanals und einem Temperatursensor am Gehäuse. Eine zu lösende Aufgabe besteht darin, den Feuchtigkeitsgehalt eines strömenden Mediums auf möglichst einfache Weise zu bestimmen.

Es wird eine Messvorrichtung angegeben, die einen Feuchtigkeitssensor zur Erfassung der Feuchtigkeit eines strömenden Mediums aufweist. Der Feuchtigkeitssensor ist auf einem Träger angeordnet. Außerden befindet sich auf dem Träger ein Temperatursensor, der die Temperatur des Mediums erfassen kann.

Erfindungsgemäß weist der Träger strömungslenkende Elemente auf, die dazu geeignet sind, das strömende Medium in Richtung eines oder beider Sensoren zu lenken.

Hierbei weist der Träger einen Kanal mit einer Einlassöffnung und einer Auslassöffnung für das strömende Medium auf. Innerhalb des Kanals können sich die strömungslenkenden Elemente z. B. in Form von Umlenkflächen oder rillenartigen Vertiefungen befinden.

In einer bevorzugten Ausführungsform sind die strömungslenkenden Elemente so ausgebildet und angeordnet, dass der Feuchtigkeitssensor die mittlere Feuchtigkeit des Mediums erfassen kann.

Die strömungslenkenden Elemente bewirken eine Konzentration des strömenden Mediums in Richtung des Sensors. Dies bedeutet, dass verschiedene Teilströme, insbesondere die am Rand des Stroms verlaufenden Teile, des strömenden Mediums zusammengeführt und in Richtung des Sensors geleitet werden.

Erfindungsgemäß kann eine Konzentration des strömenden Mediums durch die Verjüngung des Querschnitts eines Kanals entlang der Strömungsrichtung des Mediums herbeigeführt werden. Das Medium strömt dabei durch die Einlassöffnung in den Kanal ein und wird von den strömungslenkenden Elementen zu einem verengten Bereich hin geleitet, dessen Querschnitt geringer ist, als der Querschnitt der Einlassöffnung. Dies führt dazu, dass Teilströme des Mediums zusammengeführt werden. In einer Ausführungsform wird das Medium durch die Verengung des Kanals komprimiert und trifft in komprimierter Form auf den Feuchtigkeitssensor. Aus der gemessenen Feuchtigkeit des Mediums in seiner komprimierten Form kann die mittlere Feuchtigkeit des Mediums in seiner unkomprimierten Form hergeleitet werden.

Der Kanal kann trichterförmig ausgebildet sein. Beispielsweise befindet sich der Feuchtigkeitssensor im Bereich des Trichterhalses, in dem das strömende Medium konzentriert vorliegt.

Zusätzlich zur Konzentration des Mediums können die strömungslenkenden Elemente eine Durchmischung eines Volumens des vorbeiströmenden Mediums bewirken. Vorzugsweise tritt hier keine Komprimierung des Mediums auf.

In einer weiteren Ausführungsform sind die strömungslenkenden Elemente und der Feuchtigkeitssensor so angeordnet, dass das Medium in Richtung des Feuchtigkeitssensors gelenkt wird, ohne dass eine Konzentration oder Durchmischung erfolgt. Dann kann der Feuchtigkeitssensor punktuell die Feuchtigkeit des Mediums bestimmen. Hierbei ist mit dem Begriff "punktuell" gemeint, dass die Feuchtigkeit des Mediums in seiner unkonzentrierten Form an einer Stelle erfasst wird.

Dazu ist der Feuchtigkeitssensor in einem Bereich der Messvorrichtung angeordnet, in dem das strömende Medium nicht konzentriert und nicht durchmischt vorliegt. Beispielsweise ist der Feuchtigkeitssensor in einem nicht verengten Bereich eines Kanals angeordnet, in dem der Querschnitt gleich dem Querschnitt der Einlassöffnung ist. Der Feuchtigkeitssensor kann auch direkt am Einlass des Kanals angeordnet sein.

Der Temperatursensor kann ebenso wie der Feuchtigkeitssensor so angeordnet werden, dass er eine mittlere oder eine punktuelle Temperatur des Mediums erfassen kann.

In einer nicht beanspruchten Ausführungsform sind der Temperatursensor und der Feuchtigkeitssensor in einem gemeinsamen Sensorelement integriert.

Bei dieser Anordnung kann in einfacher Weise an derselben Stelle die Feuchtigkeit und die Temperatur des Mediums erfasst werden.

In einer erfindungsgemäßen Ausführungsform sind der Temperatursensor und der Feuchtigkeitssensor als separate Bauteile ausgeführt. Diese Bauteile können an verschiedenen Stellen des Trägers angeordnet sein.

Bei dieser Ausführungsform kann beispielsweise der Feuchtigkeitssensor die mittlere Feuchtigkeit des Mediums bestimmen, während der Temperatursensor eine punktuelle Temperatur des Mediums erfasst. Dazu ist der Feuchtigkeitssensor in einem Bereich des Trägers angeordnet, in dem ein konzentriertes oder durchmischtes Volumen des Mediums strömt. Der Temperatursensor kann in einem Bereich des Trägers angeordnet sein, in dem das vorbeiströmende Medium nicht konzentriert und nicht durchmischt ist. Beispielsweise ist der Temperatursensor am Einlass eines Kanals angeordnet und der Feuchtigkeitssensor in einem verengten Bereich des Kanals, der einen geringeren Querschnitt als die Einlassöffnung aufweist.

In einer alternativen Ausführungsform sind der Feuchtigkeitssensor und der Temperatursensor so angeordnet, dass die Feuchtigkeit des Mediums punktuell und die Temperatur des Mediums über ein Volumen gemittelt erfasst wird.

Die Vorrichtung kann zudem Elemente aufweisen, die ein Wärme leitendes Material enthalten. Diese Elemente sind so in der Messvorrichtung angeordnet, dass sie vom strömenden Medium wenigstens teilweise umströmt oder durchströmt werden. Die Wärmeleitfähigkeit des Materials sollte dabei so groß sein, das sich zumindest in einem Teilbereich des Elementes innerhalb kurzer Zeit eine mittlere Temperatur einstellt, die der mittleren Temperatur des strömenden Mediums entspricht oder aus der sich die mittlere Temperatur des Mediums in einfacher Weise herleiten lässt. Beispielsweise ragt das Element in einen Kanal der Messvorrichtung hinein und wird vom Medium teilweise umströmt. In einer Ausführungsform ist das Element als Gitter ausgebildet, das sich in einem Kanal der Messvorrichtung befindet und sich über den Querschnitt des Kanals erstreckt.

Vorzugsweise ist der Temperatursensor mit diesem Element thermisch verbunden und erfasst die Temperatur des Elementes. Dadurch kann in einfacher Weise eine mittlere Temperatur des Mediums ermittelt werden, ohne dass das Medium konzentriert oder durchmischt werden muss. Zudem muss der Temperatursensor dann nicht mehr in einem Bereich der Messvorrichtung angeordnet sein, an dem das Medium direkt vorbeiströmt und ist so besser vor mechanischer Beschädigung geschützt.

Vorzugsweise sind in der Messvorrichtung Elemente zur Abschirmung des Feuchtigkeitssensors oder des Temperatursensors vorgesehen. Diese Elemente können beispielsweise an einer Einlassöffnung der Messvorrichtung, oberhalb des Sensorelementes angeordnet sein.

Die Abschirmelemente verhindern, dass das Medium direkt auf das Sensorelement auftrifft. Dies dient zum Einen dem Schutz des Sensorelements. Das Abschirmelement kann zudem sicherstellen, dass eine Konzentration oder Vermischung des Mediums auftritt, bevor dieses auf das Sensorelement trifft. Dies ist insbesondere bei der Bestimmung der mittleren Feuchtigkeit oder der mittleren Temperatur von Vorteil.

In einer bevorzugten Ausführungsform ist ein Sensorelement mit einem elektronischen Schaltkreis verbunden, der ein Sensorsignal in andere elektronische Signale, beispielsweise Strom, Spannung, Frequenz oder digitale Protokolle, umwandelt. Dazu ist das Sensorelement beispielsweise auf einer Leiterplatte angeordnet und mit dem elektronischen Schaltkreis verbunden.

Der elektronische Schaltkreis ist vorzugsweise so ausgebildet, dass eine Kalibrierung des Sensorelements durchgeführt werden kann. Trifft das Medium als komprimiertes Volumen auf den Feuchtigkeitssensor auf, so kann beispielsweise durch eine geeignete Kalibrierung der korrekte Wert für die mittlere Feuchtigkeit des Mediums in seiner unkomprimierten Form ausgegeben werden.

In einer bevorzugten Ausführungsform ist das strömende Medium ein strömendes Gas, insbesondere Luft.

Im Folgenden werden die angegebene Messvorrichtung und ihre vorteilhaften Ausgestaltungen anhand von schematischen und nicht maßstabsgetreuen Figuren erläutert. Es zeigen:
- Figur 1: im Querschnitt eine Messvorrichtung mit einem Feuchtigkeitssensor und einem darin integrierten Temperatursensor,
- Figur 2: im Querschnitt eine Messvorrichtung mit einem Feuchtigkeitssensor und einem separaten Temperatursensor,
- Figur 3: im Querschnitt eine Messvorrichtung mit einem Feuchtigkeitssensor und einem separaten Temperatursensor, der von einem Element aus einem wärmeleitenden Material umgeben ist.

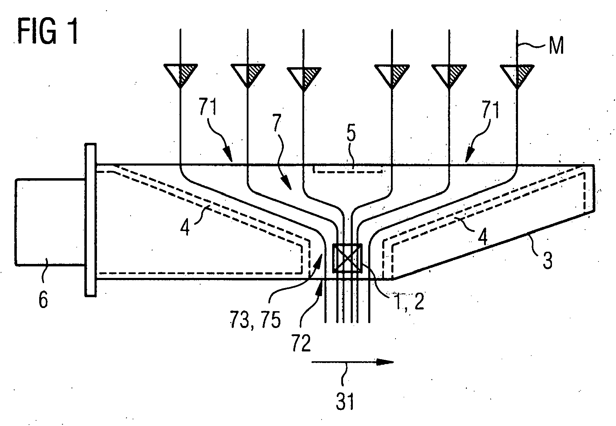

Figur 1 zeigt eine nicht beanspruchte Messvorrichtung, die von einem strömenden Medium M durchströmt wird. Die Messvorrichtung weist einen Träger 3 auf, der mit seiner Längsachse 31 senkrecht zur Strömungsrichtung (Pfeile) des strömenden Mediums M ausgerichtet ist. In der Messvorrichtung befindet sich ein Kanal 7 mit einer Einlassöffnung 71 und einer Auslassöffnung 72. Im Kanal ist ein Feuchtigkeitssensor 1 angeordnet, in den ein Temperatursensor 2 integriert ist. Das strömende Medium M strömt durch die Einlassöffnung 71 in die Messvorrichtung ein, an den Sensoren 1, 2 vorbei und durch die Auslassöffnung 72 aus der Messvorrichtung heraus.

Das Medium M wird mittels strömungslenkender Elemente 4 in Richtung der Sensoren 1, 2 konzentriert. Die strömungslenkenden Elemente 4 sind als Umlenkflächen ausgebildet, die gegen die Längsachse 31 des Trägers 3 verkippt sind. Sie bilden einen Trichter, in dessen Hals 75 sich die Sensoren 1, 2 befinden. Durch die trichterförmige Anordnung wird das einströmende Medium M in Richtung der Sensoren 1, 2 gelenkt. Da sich der Trichter in Richtung des Sensorelementes 1, 2 verjüngt, werden Teilströme des Mediums M in Richtung des Trichterhalses 75 zusammengeführt und vorzugsweise vermischt. Somit können die Sensoren 1, 2, die sich in einem verengten Bereich 73 des Kanals 7 befinden, die mittlere Feuchtigkeit und die mittlere Temperatur des Mediums M erfassen.

An der Einlassöffnung 71 des Kanals 7 ist ein Abschirmelement 5 angeordnet, das die Sensoren 1, 2 abschattet. Das Abschirmelement 5 verhindert, dass das einströmende Medium M direkt auf die Sensoren 1, 2 einströmt.

Die Messvorrichtung kann mittels eines Steckers 6 elektrisch angeschlossen werden. Der Stecker 6 steht beispielsweise mittels einer Leiterplatte mit den Sensoren 1, 2 in elektrischem Kontakt. Vorzugsweise werden beim elektrischen Anschluss der Messvorrichtung auch die erfassten Messdaten ausgegeben und weiterverarbeitet.

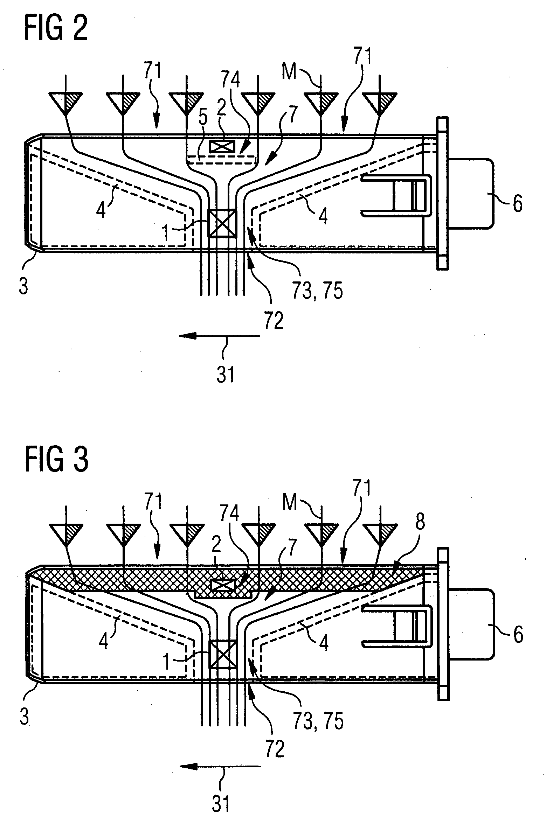

Figur 2 zeigt eine erfindungsgemäße Messvorrichtung, bei der der Feuchtigkeitssensor 1 und der Temperatursensor 2 in verschiedenen Bereichen eines Kanals 7 angeordnet sind. Der Kanal 7 ist hier wie in Figur 1 trichterförmig ausgebildet. Der Feuchtigkeitssensor 1 ist wieder im Trichterhals 75 angeordnet und dient zur Bestimmung der mittleren Feuchtigkeit des strömenden Mediums M. Der Feuchtigkeitssensor 1 wird durch ein Abschirmelement 5 vor einer direkten Einströmung des Mediums M geschützt. Der Temperatursensor 2 befindet sich auf der dem Feuchtigkeitssensor 1 gegenüberliegenden Seite des Abschirmelements 5 und wird direkt vom Medium M angeströmt. Der Temperatursensor 2 ist auf der Höhe der Einlassöffnung 71 angeordnet. In diesem nicht verengten Bereich 74 liegt das Medium M nicht konzentriert oder durchmischt vor. Somit kann an dieser Stelle eine punktuelle Temperatur des strömenden Mediums M bestimmt werden, d. h. die Temperatur ist nicht über ein Volumen gemittelt.

Figur 3 zeigt eine erfindungsgemäße Messvorrichtung mit einem trichterförmigen Kanal 7, bei der der Feuchtigkeitssensor 1 im Trichterhals 75 angeordnet ist. Der Temperatursensor 2 befindet sich im Bereich der Einlassöffnung 71 und ist von einem Gitter 8 umgeben, das ein wärmeleitendes Material enthält. Das Gitter 8 erstreckt sich über den Querschnitt der Einlassöffnung 71 des Kanals 7 und wird vom strömenden Medium M durchströmt. Dabei erwärmt sich das Gitter 8. Aufgrund der guten Wärmeleitfähigkeit des Gitters 8 stellt sich innerhalb kurzer Zeit im Bereich des Temperatursensors 2 eine mittlere Temperatur ein. Der Temperatursensor 2 misst diese Temperatur und kann daraus die über den Querschnitt der Einlassöffnung 71 gemittelte Temperatur des strömenden Mediums M bestimmen. Das Gitter 8 bewirkt zudem eine Durchmischung des Mediums M und stellt damit auch ein strömungslenkendes Element dar. Vorzugsweise ist ein Abschirmelement 5 in das Gitter 8 integriert, das den Feuchtigkeitssensor 1 und den Temperatursensor 2 abschattet.

In einer alternativen, nicht beanspruchten Ausführungsform kann der Temperatursensor 2 auch außerhalb des Kanals 7 angeordnet sein, so dass er nicht mit dem strömenden Medium M in Kontakt kommt.

### Bezugszeichenliste

- 1: Feuchtigkeitssensor
- 2: Temperatursensor
- 3: Träger
- 31: Längsachse des Trägers
- 4: strömungslenkendes Element
- 5: Abschirmelement
- 6: Stecker
- 7: Kanal
- 71: Einlassöffnung
- 72: Auslassöffnung
- 73: verengter Bereich
- 74: nicht verengter Bereich
- 75: Trichterhals
- 8: Element aus wärmeleitendem Material
- M: strömendes Medium

## Patentansprüche

1. Messvorrichtung
- mit einem Feuchtigkeitssensor (1) zur Erfassung der Feuchtigkeit eines strömenden Mediums (M),
- mit einem Temperatursensor (2) zur Erfassung der Temperatur des strömenden Mediums (M),
- wobei der Feuchtigkeitssensor (1) und der Temperatursensor (2) auf einem gemeinsamen Träger (3) angeordnet sind und
- wobei der Träger (3) strömungslenkende Elemente (4, 5) aufweist.
- wobei die strömungslenkenden Elemente (4, 5) zur Konzentration des strömenden Mediums (M) in Richtung des einen Sensors geeignet sind, wobei verschiedene Teilströme des strömenden Mediums zusammengeführt und in Richtung dieses Sensors geleitet werden,
- wobei der Träger (3) einen Kanal (7) aufweist mit einer Einlassöffnung (71), durch die das strömende Medium (M) in den Kanal (7) einströmt,
- wobei der Kanal (7) einen verengten Bereich (73) aufweist, dessen Querschnitt geringer ist als der Querschnitt der Einlassöffnung (71),
- wobei der eine Sensor aus der Menge von Feuchtigkeitssensore (1) und Temperatursensor (2) im verengten Bereich (73) angeordnet ist, **gekennzeichnet dadurch**
- **dass** der Kanal (7) einen nicht verengten Bereich (74) aufweist, dessen Querschnitt gleich dem Querschnitt der Einlassöffnung (71) ist, und
- wobei der andere Sensor aus der Menge von Feuchtigkeitssensor (1) und Temperatursensor (2) im nicht verengten Bereich (74) angeordnet ist.

2. Messvorrichtung nach Anspruch 1,
- bei der die strömungslenkenden Elemente (4, 5) zur Lenkung des strömenden Mediums (M) in Richtung wenigstens eines Sensors aus der Menge von Feuchtigkeitssensor (1) und Temperatursensor (2) geeignet sind.

3. Messvorrichtung nach einem der Ansprüche 1 oder 2,
- bei der die strömungslenkenden Elemente (4, 5) zur Durchmischung des strömenden Mediums (M) geeignet sind.

4. Messvorrichtung nach einem der vorherigen Ansprüche,
- bei der der Kanal (7) trichterförmig ausgebildet ist und einen Trichterhals (75) aufweist.

5. Messvorrichtung nach Anspruch 4,
- bei der der Feuchtigkeitssensor (2) im Bereich der Einlassöffnung (71) und der Temperatursensor (1) im Bereich des Trichterhalses (75) angeordnet ist.

6. Messvorrichtung nach Anspruch 4,
- bei der der Temperatursensor (1) im Bereich der Einlassöffnung (71) und der Feuchtigkeitssensor (2) im Bereich des Trichterhalses (75) angeordnet ist.

7. Messvorrichtung nach einem der Ansprüche 1 bis 6,
- mit einem Element (8), das ein wärmeleitendes Material enthält, und das mit dem Temperatursensor (2) thermisch verbunden ist.

8. Messvorrichtung nach Anspruch 7,
- bei dem das Element (8) ein Gitter ist, das vom strömenden Medium (M) durchströmt wird.

9. Messvorrichtung nach einem der Ansprüche 1 bis 8,
- mit einem Abschirmelement (5), das eine direkte Einströmung des strömenden Mediums (M) auf wenigstens einen Sensor aus der Menge von Feuchtigkeitssensor (1) und Temperatursensor (2) verhindert.

10. Messvorrichtung nach einem der Ansprüche 1 bis 9,
- die zur Messung der Feuchtigkeit und der Temperatur von durchströmender Luft dient.

## Claims

1. Measuring device
- having a moisture sensor (1) for detecting the amount of moisture in a flowing medium (M),
- having a temperature sensor (2) for detecting the temperature of the flowing medium (M),
- the moisture sensor (1) and the temperature sensor (2) being arranged on a common carrier (3), and
- the carrier (3) having flow-directing elements (4, 5),
- the flow-directing elements (4, 5) being suitable for concentrating the flowing medium (M) in the direction of one of the sensors, different partial flows of the flowing medium being combined and led in the direction of said sensor,
- the carrier (3) having a channel (7) having an inlet opening (71) through which the flowing medium (M) flows into the channel (7),
- the channel (7) having a constricted region (73) whose cross section is smaller than the cross section of the inlet opening (71),
- one of the sensors from the set of moisture sensor (1) and temperature sensor (2) being arranged in the constricted region (73), **characterized**
- **in that** the channel (7) has a non-constricted region (74) whose cross section is equal to the cross section of the inlet opening (71), and
- the other sensor from the set of moisture sensor (1) and temperature sensor (2) being arranged in the non-constricted region (74).

2. Measuring device according to Claim 1,
- in which the flow-directing elements (4, 5) are suitable for directing the flowing medium (M) in the direction of at least one sensor from the set of moisture sensor (1) and temperature sensor (2).

3. Measuring device according to either of Claims 1 and 2,
- in which the flow-directing elements (4, 5) are suitable for mixing the flowing medium (M).

4. Measuring device according to one of the preceding claims,
- in which the channel (7) is in the form of a funnel and has a funnel neck (75).

5. Measuring device according to Claim 4,
- in which the moisture sensor (2) is arranged in the region of the inlet opening (71) and the temperature sensor (1) is arranged in the region of the funnel neck (75).

6. Measuring device according to Claim 4,
- in which the temperature sensor (1) is arranged in the region of the inlet opening (71) and the moisture sensor (2) is arranged in the region of the funnel neck (75).

7. Measuring device according to one of Claims 1 to 6,
- having an element (8) which contains a thermally conductive material and is thermally connected to the temperature sensor (2).

8. Measuring device according to Claim 7,
- in which the element (8) is a grating through which the flowing medium (M) flows.

9. Measuring device according to one of Claims 1 to 8,
- having a shielding element (5) which prevents the flowing medium (M) from flowing directly onto at least one sensor from the set of moisture sensor (1) and temperature sensor (2).

10. Measuring device according to one of Claims 1 to 9,
- which is used to measure the amount of moisture in and the temperature of air flowing through.

## Revendications

1. Dispositif de mesure
- avec un détecteur d'humidité (1) pour détecter l'humidité d'un fluide en écoulement (M),
- avec un capteur de température (2) pour détecter la température du fluide en écoulement (M),
- dans lequel le détecteur d'humidité (1) et le capteur de température (2) sont disposés sur un support commun (3) et
- dans lequel le support (3) présente des éléments de déviation d'écoulement (4, 5),
- dans lequel les éléments de déviation d'écoulement (4, 5) conviennent pour la concentration du fluide en écoulement (M) en direction d'un détecteur, dans lequel différents courants partiels du fluide en écoulement sont rassemblés et guidés en direction de ce détecteur,
- dans lequel le support (3) présente un canal (7) avec une ouverture d'entrée (71), par laquelle le fluide en écoulement (M) pénètre dans le canal (7),
- dans lequel le canal (7) présente une zone rétrécie (73), dont la section transversale est plus petite que la section transversale de l'ouverture d'entrée (71),
- dans lequel ledit un détecteur du groupe du détecteur d'humidité (1) et du capteur de température (2) est disposé dans la zone rétrécie (73),
**caractérisé en ce que**
- le canal (7) présente une zone non rétrécie (74), dont la section transversale est égale à la section transversale de l'ouverture d'entrée (71), et
- dans lequel l'autre détecteur du groupe du détecteur d'humidité (1) et du capteur de température (2) est disposé dans la zone non rétrécie (74).

2. Dispositif de mesure selon la revendication 1,
- dans lequel les éléments de déviation d'écoulement (4, 5) conviennent pour la déviation du fluide en écoulement (M) en direction d'au moins un détecteur du groupe du détecteur d'humidité (1) et du capteur de température (2).

3. Dispositif de mesure selon une des revendications 1 ou 2,
- dans lequel les éléments de déviation d'écoulement (4, 5) conviennent pour le mélange intime du fluide en écoulement (M).

4. Dispositif de mesure selon l'une quelconque des revendications précédentes,
- dans lequel le canal (7) est réalisé en forme d'entonnoir et présente un col d'entonnoir (75).

5. Dispositif de mesure selon la revendication 4,
- dans lequel le détecteur d'humidité (2) est disposé dans la région de l'ouverture d'entrée (71) et le capteur de température (1) est disposé dans la région du col d'entonnoir (75).

6. Dispositif de mesure selon la revendication 4,
- dans lequel le capteur de température (1) est disposé dans la région de l'ouverture d'entrée (71) et le détecteur d'humidité (2) est disposé dans la région du col d'entonnoir (75).

7. Dispositif de mesure selon l'une quelconque des revendications 1 à 6,
- avec un élément (8), qui contient un matériau conducteur de la chaleur, et qui est thermiquement relié au capteur de température (2).

8. Dispositif de mesure selon la revendication 7,
- dans lequel l'élément (8) est une grille, qui est traversée par le fluide en écoulement (M).

9. Dispositif de mesure selon l'une quelconque des revendications 1 à 8,
- avec un élément de protection (5), qui empêche un impact direct du fluide en écoulement (M) sur au moins un détecteur du groupe du détecteur d'humidité (1) et du capteur de température (2).

10. Dispositif de mesure selon l'une quelconque des revendications 1 à 9,
- qui est utilisé pour la mesure de l'humidité et de la température d'air en circulation.
